(19) Europäisches Patentamt
European Patent Office
Office européen des brevets

(11) Veröffentlichungsnummer: **0 233 337 B1**

(12) **EUROPÄISCHE PATENTSCHRIFT**

(45) Veröffentlichungstag der Patentschrift: **27.02.91**

(51) Int. Cl.5: **C07C 45/58**

(21) Anmeldenummer: **86116806.0**

(22) Anmeldetag: **03.12.86**

# (54) Verfahren zur Herstellung von Carbonylverbindungen.

(30) Priorität: **18.01.86 DE 3601380**

(43) Veröffentlichungstag der Anmeldung:
**26.08.87 Patentblatt 87/35**

(45) Bekanntmachung des Hinweises auf die Patenterteilung:
**27.02.91 Patentblatt 91/09**

(84) Benannte Vertragsstaaten:
**AT CH DE ES FR GB IT LI NL SE**

(56) Entgegenhaltungen:
**DE-A- 3 136 886**
**US-A- 4 517 386**

**RESEARCH DISCLOSURE, Nr. 115, November 1973, Seiten 5-6, Nr. 11504; "Isomerization of epoxy compounds"**

(73) Patentinhaber: **Degussa Aktiengesellschaft**
**Weissfrauenstrasse 9**
**W-6000 Frankfurt am Main 1(DE)**

(72) Erfinder: **Berg, Marion**
**Kornblumenweg 1**
**D-6458 Rodenbach 1(DE)**
Erfinder: **Grund, Andreas, Dr.**
**Rilkeweg 15**
**D-6100 Darmstadt(DE)**
Erfinder: **Prescher, Günther, Dr.**
**Liesingstrasse 2**
**D-6450 Hanau 9(DE)**

**EP 0 233 337 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von gegebenenfalls substituierten Carbonylverbindungen durch Umlagerung von Epoxyverbindungen.

Die bei der Isomerisierung von Epoxiden erhaltenen Carbonylverbindungen sind wertvolle Zwischenprodukte für organische Synthesen und interessante Rohprodukte für die chemische Industrie, z.B. für Kosmetika oder Pharmazeutika und Agrochemikalien.

Auf Grund dieser Tatsache wurde die Isomerisierung häufiger untersucht, z.B. mit sauren oder basisch wirkenden Katalysatoren, wie z.B.Bortrifluorid oder tert. Kaliumbutylat.
Mit sauren Katalysatoren werden Gemische von Ketonen und Aldehyden, mit basischen Katalysatoren hauptsächlich ungesättigte Alkohole gebildet.

Die Zusammensetzung der Gemische aus Aldehyden und Ketonen schwankte stark. So konnten zwar Epoxyfettsäureester mit Bortrifluoridätherat unter Verwendung von siedendem Dioxan zu 70 - 90 % zu Ketofettsäureestern umgelagert werden. (J.Am. Oil Chem. Soc. 42 [1965] , 126 ff.) Technisch war dieses Verfahren aber äußerst problematisch durchzuführen wegen des leicht brennbaren Dioxans, das dazu noch in großen Mengen eingesetzt werden mußte.
Außerdem hat Bortrifluoridätherat den Nachteil, daß es stark korrodierend wirkt und völlig wasserfreie Lösungsmittel und Einsatzprodukte erfordert.

Bei einem jüngeren Verfahren, bei dem die Isomerisierung von Oxiranen mit sauren Katalysatoren, nämlich Halogenwasserstoffen oder deren Umsetzungsprodukten in wäßrigem Medium durchgeführt wird, werden nur dann etwas höhere Ausbeuten an Ketoverbindungen in dem Gemisch, d.h. oberhalb 50 %, erhalten, wenn das Abdestillieren des eingeführten Wassers unter Inertgas erfolgt.

Ohne Verwendung des Inertgases werden Ausbeuten am Gemisch "Aldehyd - Keton" unterhalb 50 % gewonnen (DE-OS 33 34 600). Auch in dem Verfahren der US-PS 4,451,672 mußte bei Verwendung von Dicobaltcarbonyl in tert. Butylalkohol unter Argon gearbeitet werden.

Ebenso haben die mit anorganischen Metallsalzen arbeitenden Verfahren wie die der japanischen Anmeldungen 72/40779 und 81/047442 für die technische Durchführung schwerwiegende Nachteile. Die Entfernung der in dem Reaktionsgemisch enthaltenen Katalysatoren gestaltet sich sehr aufwendig.

Technisch nicht durchführbar wegen der Gefährlichkeit der eingesetzten Substanzen waren auch Methoden, die mit Lithiumbromid oder - perchlorat in Gegenwart von Phosphinoxiden oder Phosphorsäuretrisamid in Benzol arbeiteten (J. Am. Chem. Soc. 93 [1971] , 1963 ).

Bekannt ist auch die Verwendung von Natriumjodid zusammen mit Propyljodid. Hierbei wurde als Lösungsmittel Dimethylsulfoxid verwendet (J. Chem. Soc. Chem. Comm. [1968] , 227 ff.) Das Katalysatorgemisch mußte in fünffachem Überschuß eingesetzt werden. Hinzu kam, daß die Isolierung der Substanz aus dem Reaktionsgemisch wegen des ausgefallenen Jods schwierig war. Die eingesetzte Katalysatormischung ließ sich außerdem nur für eine einzige Umsetzung verwenden, da sie mit Wasser ausgewaschen wurde.

Demgegenüber führte das Verfahren der DE-OS 31 36 886 zu einem Ketongehalt des Aldehyd-Ketongemisches von mindestens 75 % unter Verwendung von Natriumjodid allein in Gegenwart von Dimethylsulfoxid oder Dimethylformamid. Die Ausbeuten an Keton lagen ebenfalls über 70 %. Der eingesetzte Katalysator ließ sich ohne aufwendiges Aufarbeiten mehrmals einsetzen. Das gewonnene Keton mußte jedoch durch wäßrige Extraktion aus Dimethylsulfoxid oder Dimethylformamid gewonnen werden. Das eingesetzte Lösungsmittel ging dabei verloren.

Zweck der Anmeldung ist ein technisch durchführbares Verfahren zur Herstellung von Carbonylverbindungen, vor allem Ketonen, durch Isomerisierung der entsprechenden Epoxide, bei dem die Ausbeute an Reaktionsgemisch, vor allem auch sein Gehalt an Keton, mindestens gleich, bevorzugt aber weit höher als nach den bekannten Verfahren und dessen Durchführung wesentlich vereinfacht ist.

Es wurde nun gefunden, daß sich diese Aufgabe lösen läßt, wenn man die Isomerisierung von Oxiranen der allgemeinen Formel

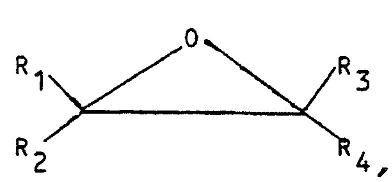

I,

in der $R_1$ und $R_3$ Wasserstoff; $R_2$ Alkyl- oder Alkylengruppen mit 3 - 26 C-Atomen oder die Phenylgruppe,

die auch substituiert sein können, und $R_4$ Wasserstoff oder Alkylgruppen mit 1 - 26 C-Atomen bedeuten und in denen die Kohlenstoffatome des Epoxidringes gegebenenfalls Bestandteile eines cyclischen Systems sind,zu Carbonylverbindungen, vor allem Ketonen, mit Hilfe eines Katalysatorsystems aus Alkalijodid und einem Polyäthylenglykol der Formel

$$HO-(CH_2-CH_2-O)_n\,H, \qquad II,$$

dessen mittlere Molmasse 400 - 10 000 beträgt, durchführt.
Die Isomerisierung wird bei Temperaturen von 120 -250 °C, vorzugsweise 170 - 200 °C, durchgeführt. Die obengenannten Oxirane sind als solche bekannt.

Von den Epoxyalkanen erwiesen sich die α - oder 1,2-Epoxyalkane als besonders günstig, unter ihnen Epoxydecan, Epoxydodecan, Epoxytetradecan; Epoxyhexadecan;1,2-Epoxy-3,3-dimethylbutan.

Als sehr gut hatten sich auch Oxirane erwiesen, in denen die beiden Kohlenstoffatome des Epoxidringes Bestandteile eines cyclischen Systems sind, d.h. Oxirane von cyclischen Olefinen, wie Cyclohexenoxid, Cycloheptenoxid, Cyclooctenoxid; 1,2-Epoxycyclododecandien-5,9; Cyclododecenoxid.

Innerhalb der einzelnen Gruppen können auch Gemische eingesetzt werden.
Auch durch die Phenylgruppe substituierte Oxirane, wie z.B. Styroloxid, oder 1-(1,2-Epoxy-propyl)-3,4-methylendioxybenzol.
Als Alkalijodide kommen Natrium- und Kaliumjodid, vor allem Natriumjodid, infrage.
Die Polyethylenglykole werden bevorzugt mit einer mittleren Molmasse von 400 - 1000, ganz besonders bevorzugt mit einer mittleren Molmasse von etwa 400 eingesetzt.

Das Katalysatorsystem aus Alkalijodid und Polyethylenglykol enthält 0,1 - 10 Gew.-% Kaliumjodid und 5 - 50 Gew.-% Polyethylenglykol, bezogen auf eingesetzte Oxirane.
Bevorzugt sind Katalysatorsysteme, die - bezogen auf eingesetzte Oxirane - 1 - 5 Gew.-% Natriumjodid und 15 - 30 Gew.-% Polyethylenglykol enthalten. Größere Zusätze an Polyethylenglykol und Natriumjodid sind unkritisch; bei zu geringen Konzentrationen fällt die Reaktionsgeschwindigkeit ab.

Als besonders günstig erwiesen sich bei der Durchführung des erfindungsgemäßen Verfahrens Polyethylenglykole, deren Siedepunkte oberhalb derer der herzustellenden Carbonylverbindungen, vor allem des herzustellenden Ketons, lagen. Hierdurch lassen sich die Carbonylverbindungen als leichter siedende Komponente destillativ aus dem Reaktionsgemisch entfernen. Das Katalysatorsystem aus Natriumjodid und Polyethylenglykol bleibt als Rückstand zurück, der ohne jede Aufarbeitung sofort wieder eingesetzt werden kann.

Dies ist ein wesentlicher Fortschritt gegenüber dem Stand der Technik, nach dem entweder der Katalysator verloren war oder nur nach mühsamer Aufarbeitung wieder eingesetzt werden konnte
Weiterhin fallen oftmals wäßrige Abfälle an, die z.T. Schwermetallsalze enthalten und aufwendig entsorgt werden müssen, siehe japanische Anmeldungen 72/40779 und 81/047442.
Es war nicht vorherzusehen, daß sich Epoxide bei Temperaturen oberhalb 100 °C in Gegenwart hydroxylgruppenhaltiger Komponenten umsetzen ließen und Ringöffnungsprodukte durch Hydroxylgruppen nur in sehr geringen Maße auftraten. Außerdem entstanden bei der Isomerisierung von α-Oxiranen überraschenderweise keine Produktgemische gemäß

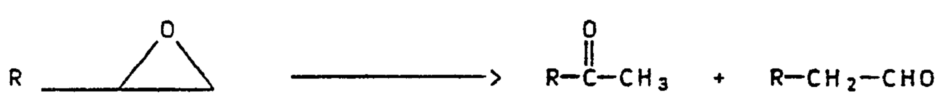
III,

nämlich Methylketon und Aldehyd, sondern es fiel fast ausschließlich das betreffende Methylketon an. Die Ausbeute an diesem letzteren betrug in der Regel mindestens 80 - 90 %. Das erfindungsgemäße Verfahren eignet sich daher besonders gut zur Herstellung von Pinakolon aus 1,2-Epoxy-3,3-dimethylbutan.

Da nach dem Verfahren der US-PS 4,517,386 bei der Umsetzung von α-Oxiranen mit Lithiumtetrafluorborat in Gegenwart von Polyäthern, d.h. Komponenten ohne Hydroxylgruppen, Gemische mit fast ausschließlichem Gehalt an Aldehyden entstanden, war das vorliegende Ergebnis völlig unerwartet; besonders, da nach dem letztgenannten Verfahren nur recht mäßige Ausbeuten erhalten wurden.

Bei der Verwendung von cyclischen Alkenoxiden bildet sich in hoher Ausbeute das Cycloalkanon

gemäß folgendem allgemeinem Reaktionsschema:

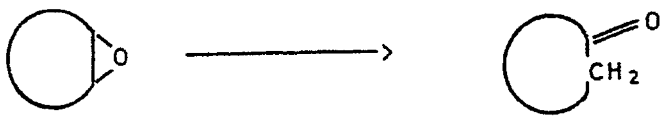

IV.

Die Ausbeuten liegen über 80 %, Ringverengungsprodukte, wie sie von K. Arata, Catal. Rev. Sci. Eng. 25 (3), 365 (1983) als Hauptprodukt beschrieben werden, treten, wenn überhaupt, nur in vernachlässigbarem Maß auf.

Außer α-Oxiranen und cyclischen Oxiranen kommen auch lineare Oxirane mit innenständigen Epoxygruppen infrage.

Bei der Herstellung der einzusetzenden Epoxide kann von Monoolefinfraktionen ausgegangen werden, die Verbindungen mit unterschiedlichen Kettenlängen enthalten. Der oben angegebenen Formel I entsprechend entstehen dann Gemische aus Epoxyverbindungen mit 8 bis 28 Kohlenstoffatomen.

Bei Oxiranen mit innenständiger Epoxygruppe kann diese Epoxygruppe willkürlich verteilt sein entlang der Kohlenstoffketten, die im Oxirangemisch vorliegen.

Zur Durchführung des erfindungsgemäßen Verfahrens werden das Ausgangsmaterial mit dem Polyethylenglykol und dem Alkalijodid in Kontakt gebracht und bei den oben angegebenen Temperaturen längere Zeit, z.B. 2 - 10 Stunden, gerührt. Der Fortgang der Reaktion läßt sich leicht durch bekannte Methoden, wie z.B. durch Gaschromatografie, bestimmen.

Die Verwendung eines Inertgases ist nicht nötig.

Das erfindungsgemäße Verfahren läßt sich unter verschiedenen Drucken durchführen, u.a. wird unter Normaldruck gearbeitet; das Verfahren läßt sich aber auch bei Über- oder Unterdruck durchführen. Wesentlich ist nur, daß die Reaktionstemperatur erreicht wird.

Die Umsetzung kann sowohl diskontinuierlich als auch kontinuierlich in für diese Art geeigneten Reaktoren, wie Rührkesseln, Kaskaden, Rohr- oder Schlaufenreaktoren, erfolgen, wobei die Reaktionswärme auf beliebige Weise zu- bzw. abgeführt wird.
Geeignete Werkstoffe für die Reaktionsapparate sind z.B. Glas, Edelstahl oder emailliertes Material.

Der technische Fortschritt liegt - wie schon angegeben wurde - in der technisch einfachen Durchführung des Verfahrens, bei dem das Gemisch nach Beendigung der Reaktion destillativ in das über Kopf abgehende Reaktionsprodukt und das als Rückstand anfallende Katalysatorsystem aufgetrennt wird.

Trotz der Vereinfachung des Isomerisierungsverfahrens ist die Ausbeute an Carbonylverbindungen nicht nur mindestens so hoch wie bei den bekannten Verfahren, sondern fast immer wesentlich höher.
Bei Einsetzen von α-Oxiranen wird zum ersten Mal ein Reaktionsgemisch erhalten, das fast vollständig aus dem betreffenden Methylketon besteht. Das im Sumpf angefallene Katalysatorsystem kann sofort wieder zur weiteren Herstellung der Carbonylverbindungen verwendet werden, und anschließende Reinigungsverfahren zu seiner Wiedergewinnung sind überflüssig.

Besonders vorteilhaft ist die Verwendung von Polyethylenglykolen, da diese völlig ungiftig bzw. physiologisch unbedenklich sind. Wegen des sehr hohen Flammpunktes und der problemlosen biologischen Abbaubarkeit sind sie für die erfindungsgemäße Umsetzung hervorragend geeignet.

Das erfindungsgemäße Verfahren wird in den Beispielen 1 - 14 folgendermaßen durchgeführt:

Man mischt das Epoxid (ca. 100 g) der in der Tabelle 1 angegebenen Menge Polyethylenglykol mit den Molekulargewichten 400, 600, 2000 oder 10 000 und Natriumjodid, wobei die Mengenangaben in Prozenten, bezogen auf das eingesetzte Epoxid, angeführt sind.

Das Gemisch wird unter gutem Rühren auf die angegebenen Temperaturen erhitzt, z.T. kann dabei Rückfluß auftreten. Der Umsatz des Epoxids wird per Gaschromatografie verfolgt. Nach erfolgter Umsetzung wird das Produkt destillativ, gegebenenfalls unter Vakuum, aus dem Reaktionsgemisch abgetrennt. Ein geringer Vorlauf an Leichtsiedern kann dabei auftreten.
Der Destillationsrückstand läßt sich unmittelbar für darauf folgende Umsetzungen als Katalysator verwenden.

Im Gegenbeispiel 14 wird statt Polyethylenglykol Diglyme, d.h Diethylenglykoldimethyläther, eingesetzt.

Alle Prozentangaben sind Gewichtsprozente.

Tabelle 1

| Nr. | Epoxid | Produkt | PEG | % PEG | % NaI | T [°C] | Rkt. Dauer [h] | Umsatz Epoxid [%] | Ausbeute [%] | Bemerk. |
|---|---|---|---|---|---|---|---|---|---|---|
| 1 | 1,2-Epoxydecan | 2-Decanon | PEG 400 | 30 | 6 | 185 | 5,5 | 97,3 | 94,8 | |
| 2 | 1,2-Epoxydodecan | 2-Dodecanon | PEG 400 | 30 | 6 | 191 | 3 | 99,0 | 88,7 | |
| 3 | 1,2-Epoxytetradecan | 2-Tetradecanon | PEG 2000 | 25 | 6 | 193 | 5 | 98,6 | 90,1 | |
| 4 | 1,2-Epoxyhexadecan | 2-Hexadecanon | PEG 10000 | 25 | 5 | 180 | 5,5 | 92,5 | 86,7 | |
| 5 | 1,2,7,8-Diepoxyoctan | 2,7-Octandion | PEG 400 | 30 | 6 | 155 | 7 | 99,3 | 73,1 | |
| 6 | Styroloxid | Acetophenon | PEG 600 | 20 | 4 | 144 | 4 | 62,5 | 52,4 | |
| 7 | Cycloheptenoxid | Cycloheptanon | PEG 400 | 30 | 6 | 183 | 7 | 97,4 | 92,8 | |
| 8 | Cyclooctenoxid | Cyclooctanon | PEG 400 | 30 | 5 | 190 | 8 | 99,6 | 85,2 | |
| 9 | Cyclooctenoxid | Cyclooctanon | PEG 400 | Dest.-Rückst. v.Bsp.8 | | 190 | 4,5 | 99,8 | 99,7 | |
| 10 | Cyclooctenoxid | Cyclooctanon | PEG 400 | Dest.-Rückst. v.Bsp.9 | | 191 | 4,5 | 99,7 | 87,0 | |
| 11 | 1,2-Epoxycyclododecadien-5,9 | Cyclododecadien-3,7-on-1 | PEG 400 | 40 | 3 | 195 | 9 | 99,5 | 98,7 | |
| 12 | Neohexenoxid | Pinakolon | PEG 400 | 30 | 6 | 180 | 6 | 99,6 | 85,6 | im Autoklaven |
| 13 | 1-(1,2-Epoxypropyl)-3,4-methylendioxybenzol | 3,4-Methylendioxybenzylmethylketon | PEG 2000 | 31 | 5 | 180 | 8 | 99,2 | 46,0 | |
| 14 | Cyclooctenoxid | Cyclooctanon | Diglyme | 30 | 6 | 182 | 6 | 9,6 | 9,4 | Gegenbeispiel |

PEG = Polyethylenglykol

Bei Beispiel 9 wurde der Destillationsrückstand von Beispiel 8 unmittelbar als Katalysator eingesetzt, desgleichen fand der Rückstand von Beispiel 9 bei Beispiel 10 Verwendung.

Beispiel 15

In einer 100 l-Edelstahl-Blase versetzte man 26,5 kg Cyclooctenoxid mit 8,0 kg Polyethylenglykol (■ = 400) und 1,5 kg NaI und erhitzte unter Rühren 10 h auf 185 °C Der Epoxidumsatz lag bei 99,8 %. Man kühlte den Ansatz auf ca. 100 °C ab und arbeitete destillativ im Vakuum auf. Nach einem geringen Vorlauf (1,75 kg; 57 %iges Produkt) gingen bei 96 °C/15 mm 21,8 kg Keton in einer Reinheit von 99 % über. Ausbeute: 86 %.

## Ansprüche

1. Verfahren zur Isomerisierung von Oxiranen der allgemeinen Formel

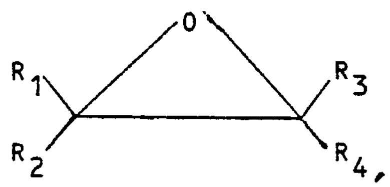

in der $R_1$ und $R_3$ Wasserstoff, $R_2$ Alkyl- oder Alkylengruppen mit 3 - 26 C-Atomen oder die Phenylgruppe, die auch substituiert sein können, und $R_4$ Wasserstoff oder Alkylgruppen mit 1 - 26 C-Atomen bedeuten, und in der die Kohlenstoffatome des Epoxydringes gegebenenfalls Bestandteile eines cyclischen Systems sind, zu Carbonylverbindungen in Gegenwart eines Katalysatorsystems bei erhöhten Temperaturen, dadurch gekennzeichnet , dass man die Isomerisierung mit Hilfe eines Katalysatorsystems aus Alkalijodid und einem Polyethylenglykol der allgemeinen Formel

$$HO - (CH_2 - CH_2 - O)_n H,$$

dessen mittlere Molmasse 400 - 10 000, vorzugsweise 400 - 1000, beträgt, bei 120 - 250 °C durchführt.

2. Verfahren nach Anspruch 1, dadurch gekennzeichnet , dass man ein Polyethylenglykol mit einer mittleren Molmasse von 400 einsetzt.

3. Verfahren nach Anspruch 1 und 2, dadurch gekennzeichnet , dass man Natriumjodid verwendet.

4. Verfahren nach Anspruch 1 bis 3, dadurch gekennzeichnet , daß man als Epoxyalkan α-oder 1,2-Epoxyalkane einsetzt.

5. Verfahren nach Anspruch 4, dadurch gekennzeichnet , daß man als α- oder 1,2-Epoxyalkan Epoxydecan, Epoxydodecan, Epoxytetradecan, Epoxyhexadecan verwendet.

6. Verfahren nach Anspruch 1 bis 4, dadurch gekennzeichnet , daß man als α-Epoxyalkan 1,2-Epoxy-3,3-dimethylbutan verwendet.

7. Verfahren nach Anspruch 1 bis 3, dadurch gekennzeichnet , daß man Cyclohexenoxid, Cycloheptenoxid, Cyclooctenoxid; 1,2-Epoxycyclododecadien-5,9; Cyclododecenoxid einsetzt.

8. Verfahren nach Anspruch 1 bis 7, dadurch gekennzeichnet , daß das Katalysatorsystem 5 - 50 Gew.-% Polyethylenglykol und 0,1 - 10 Gew.-% Natriumjodid, bezogen auf eingesetzte Oxirane, enthält.

**9.** Verfahren nach Anspruch 1 bis 8, dadurch gekennzeichnet , daß das Katalysatorsystem 15 - 30 Gew.-% Polyethylenglykol und 1 - 5 Gew.-% Natriumjodid, bezogen auf die eingesetzten Oxirane, enthält.

**10.** Verfahren nach Anspruch 1 bis 9, dadurch gekennzeichnet , daß man als Polyethylenglykole solche verwendet, deren Siedepunkte oberhalb des Siedepunktes des herzustellenden Reaktionsgemisches, vor allem des Ketons, liegen.

**Claims**

**1.** A process for the isomerisation of oxiranes corresponding to the following general formula

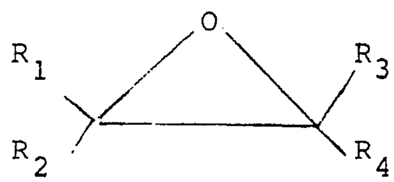

in which $R_1$ and $R_3$ stand for hydrogen, $R_2$ stands for alkyl or alkylene groups having 3 to 26 carbon atoms or the phenyl group, which groups may be substituted, and $R_4$ stands for hydrogen or alkyl groups having 1 to 26 carbon atoms, and in which the carbon atoms of the epoxide ring are optionally components of a cyclic system, to form carbonyl compounds in the presence of a catalyst system at elevated temperatures, characterised in that the isomerisation is carried out at 120 to 250 °C with the aid of a catalyst system of alkali metal iodide and a polyethylene glycol corresponding to the following general formula

$$HO - (CH_2 - CH_2 - O)_n H$$

whose average molar mass is from 400 to 10,000, preferably from 400 to 1000.

**2.** A process according to Claim 1, characterised in that a polyethylene glycol having an average molar mass of 400 is used.

**3.** A process according to Claims 1 and 2, characterised in that sodium iodide is used.

**4.** A process according to Claims 1 to 3, characterised in that the epoxyalkane used is $\alpha$- or 1,2-epoxyalkanes.

**5.** A process according to Claim 4, characterised in that the $\alpha$- or 1,2-epoxyalkane used is epoxydecane, epoxydodecane, epoxytetradecane or epoxyhexadecane.

**6.** A process according to Claims 1 to 4, characterised in that the $\alpha$-epoxyalkane used is 1,2-epoxy-3,3-dimethylbutane.

**7.** A process according to Claims 1 to 3, characterised in that cyclohexene oxide, cycloheptene oxide, cyclooctene oxide, l,2-epoxycyclodecadiene-(5,9) or cyclododecene oxide is used.

**8.** A process according to Claims 1 to 7, characterised in that the catalyst system used contains from 5 to 50% by weight of polyethylene glycol and from 0.1 to 10% by weight of sodium iodide, based on the quantity of oxirane is used.

**9.** A process according to Claims 1 to 8, characterised in that the catalyst system contains from 15 to 30% by weight of polyethylene glycol and from 1 to 5% by weight of sodium iodide, based on the quantity of oxiranes used.

**10.** A process according to Claims 1 to 9, characterised in that the polyethylene glycols used have boiling points above the boiling point of the reaction mixture to be produced, especially of the ketone.

## Revendications

**1.** Procédé d'isomérisation d'oxirannes de formule générale

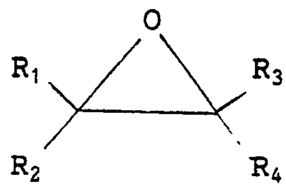

I

dans laquelle $R_1$ et $R_3$ sont l'hydrogène ; $R_2$ représente des groupes alkyles ou alkylènes de 3 à 26 atomes de C, ou le groupe phényle, qui peuvent aussi être substitués, et $R_4$ représente l'hydrogène ou des groupes alkyles avec 1-26 atomes de C, et dans lesquels les atomes de carbone du cycle époxyde font partie le cas échéant d'un système cyclique, en composés carbonyles en présence d'un système catalytique à températures élevées, caractérisé en ce qu'on réalise l'isomérisation à l'aide d'un système catalytique à base d'iodure alcalin et d'un polyéthylèneglycol de formule générale

$$HO - (CH_2 - CH_2 - O)_n H$$

dont le poids moléculaire moyen est compris entre 400 et 10 000, de préférence entre 400 et 1000, entre 120 et 250° C.

**2.** Procédé selon la revendication 1, caractérisé en ce qu'on utilise un polyéthylèneglycol d'un poids moléculaire moyen de 400.

**3.** Procédé selon les revendications 1 et 2, caractérisé en ce qu'on utilise de l'iodure de sodium.

**4.** Procédé selon les revendications 2 à 3, caractérisé en ce qu'on utilise comme époxyalcane des $\alpha$- ou 1,2-époxyalcanes.

**5.** Procédé selon la revendication 4, caractérisé en ce qu'on utilise comme $\alpha$- ou 1,2-époxyalcanes, l'époxydécane, l'époxydodécane, l'époxytétradécane, l'époxyhexadécane.

**6.** Procédé selon les revendications 1 à 4, caractérisé en ce qu'on utilise comme $\alpha$-époxyalcane le 1,2-époxy-3,3-diméthylbutane.

**7.** Procédé selon les revendications 1 à 3, caractérisé en ce qu'on utilise comme oxyde de cyclohexène, l'oxyde de cycloheptène, l'oxyde cyclooctène, le 1,2-époxycyclododécadiène-5,9, l'oxyde de cyclododécène.

**8.** Procédé selon les revendications 1 à 7, caractérisé en ce que le système catalytique contient 5 à 50 % en poids de polyéthylèneglycol et 0,1 à 10 % en poids d'iodure de sodium, par rapport aux oxirannes mis en oeuvre.

**9.** Procédé selon les revendications 1 à 8, caractérisé en ce que le système catalytique contient 15 à 30 % en poids de polyéthylèneglycol et 1-5 % en poids d'iodure de sodium, par rapport aux oxirannes mis en oeuvre.

**10.** Procédé selon les revendications 1 à 9, caractérisé en ce qu'on utilise comme polyéthylèneglycol ceux dont le point d'ébullition se situe au-dessus de celui du mélange réactionnel à préparer, avant tout de

la cétone.